# EUROPEAN PATENT APPLICATION

(11) **EP 4 424 807 A1**
(43) Date of publication of application: **04.09.2024**
(21) Application number: 22907657.5
(22) Date of filing: 17.10.2022
(51) Int. Cl.: C12M 1/00, C12M 1/34, C12M 1/36

(54) **OBJECT SUPPLYING DEVICE**

(30) Priority: 15.12.2021 KR 20210179616; 23.09.2022 KR 20220120517
(71) Applicant: Celltrio, Inc., Fremont, California 94539 (US)
(72) Inventor: KIM, Jin-Oh, Seoul 06691 (KR); HAN, Jin Seok, Seoul 05823 (KR); PAK, Seung Hoon, Gunpo-si Gyeonggi-do 15813 (KR)
(74) Representative: BCKIP Part mbB
(86) International application number: PCT/KR2022/015734
(87) International publication number: WO 2023/113186

(57) **Abstract**

Disclosed is a media liquid supply apparatus including an inflow tube part configured to define an inflow passageway through which a media liquid is introduced, moved, and discharged, a pack configured to receive the media liquid discharged from the inflow tube part, a discharge tube part configured to provide a discharge passageway through which the media liquid discharged from the pack is introduced, moved, and discharged, a tube bag part including a media pump configured to move the media liquid through the inflow passageway, and a gas injection part configured to provide an additional moving force to the media liquid moving through the inflow passageway, and a heating part configured to heat the media liquid in the pack.

## Description

### [Technical Field]

The present application relates to a media liquid supply apparatus.

### [Background Art]

In general, cell culture refers to a technology that aseptically removes tissue fragments from individuals, i.e., multicellular organisms, and cultivates and proliferates the tissue fragments in a container. Recently, in the field of cell culture and regenerative medicine, technologies have been required to automatically and stably cultivate cells in large quantities for a long period of time to obtain cell products that can be used in biologics and other products or obtain cells that can be used to build regenerative tissue.

In the related art, an operator performs the cell culture operation in the laboratory by manually transporting and manipulating flasks in which cells are cultured. However, the cell culture operation, which is manually performed by the operator, is not efficient because productivity greatly fluctuates depending on the operator's skill, and human errors, such as missing the time to change the culture solution or inject cells, cause the entire process to be discarded. In addition, because a predetermined process needs to be repeatedly performed at a predetermined time by highly paid and skilled operators, personnel costs are high, and as a result, it is difficult to reduce manufacturing costs.

Accordingly, there has been a need for a media liquid supply apparatus capable of automatically heating and discharging a media liquid stored in refrigeration.

The background art of the present application is disclosed in Korean Patent Application Laid-Open No. 10-2015-0006422.

### [Disclosure]

### [Technical Problem]

The present application has been made in an effort to solve the problems in the related art, and an object of the present application is to provide a media liquid supply apparatus capable of automatically heating and discharging a media liquid.

However, technical problems to be solved by the exemplary embodiment of the present application are not limited to the aforementioned technical problem, and other technical problems may be present.

### [Technical Solution]

As a technical solution for achieve the above-mentioned object, a media liquid supply apparatus according to an embodiment of the present application includes: a tube bag part including an inflow tube part having an inflow passageway through which a media liquid is introduced, moved, and discharged, a pack configured to receive the media liquid discharged from the inflow tube part, a discharge tube part having a discharge passageway through which the media liquid discharged from the pack is introduced, moved, and discharged, and an auxiliary tube part configured to allow the inflow tube part to communicate with the outside; a heating part configured to heat the media liquid in the pack; a dispenser configured to be supplied with the media liquid discharged through the discharge tube part and discharge the media liquid; and a pump part configured to supply the media liquid to the pack through the inflow tube part and the auxiliary tube part.

In the media liquid supply apparatus according to the embodiment of the present application, the pump part may include: a media pump configured to move a preset amount of media liquid through the inflow tube part; and a gas pump configured to guide a discharge of the media liquid to the pack by supplying a gas to prevent the media liquid from stagnating in a section between the pack and a portion of the inflow tube part where the media pump is provided.

In the media liquid supply apparatus according to the embodiment of the present application, the gas pump may be configured to supply the gas so that the media liquid in the pack moves from the pack to the discharge tube part.

In the media liquid supply apparatus according to the embodiment of the present application, the tube bag part may further include a sensor configured to measure information on the media liquid passing through the section between the portion of the inflow tube part where the media pump is provided and a portion of the inflow tube part to which the auxiliary tube part is connected.

The media liquid supply apparatus according to the embodiment of the present application may further include: a refrigeration part configured to accommodate a media bag having an internal space in which the media liquid is accommodated, in which the inflow tube part receives the media liquid from the media bag.

In the media liquid supply apparatus according to the embodiment of the present application, the media bag may be disposed at a lower side of the internal space of the refrigeration part, and the inflow tube part, the media pump, and the gas injection part may be disposed at an upper side of the internal space of the refrigeration part.

In the media liquid supply apparatus according to the embodiment of the present application, the dispenser may receive the media liquid from the discharge tube part and discharge the media liquid.

In the media liquid supply apparatus according to the embodiment of the present application, the heating part and the dispenser may be provided outside the refrigeration part.

In the media liquid supply apparatus according to the embodiment of the present application, the dispenser may include: a tube; and a fixing plate part configured to fix a position of the tube, and the discharge tube part may connect the pack and the tube and supply the media liquid, which is discharged from the pack, to the tube.

The technical solution is just illustrative but should not be interpreted as being intended to limit the present application. In addition to the above-mentioned exemplary embodiment, additional exemplary embodiments may be present in the drawings and the detailed description.

### [Advantageous Effects]

According to the technical solution of the present application, the media liquid may be introduced into the pack through the inflow tube part by the media pump, heated in the pack, and then discharged through the discharge tube part, such that the media liquid supply apparatus capable of automatically heating and discharging the media liquid may be implemented.

In addition, the media liquid may not only be introduced into the pack by the media pump, but also be introduced into the pack by being pressed by a pressure of gas supplied by a gas supply part. Therefore, it is possible to prevent the occurrence of the media liquid, which remains in the inflow tube part without being supplied to the pack, and the media liquid that remains in the pack. Therefore, it is possible to prevent the waste of the media liquid, i.e., prevent the occurrence of the discarded media liquid and minimize the amount of consumption of the media liquid.

In addition, the preset amount of media liquid may be introduced into the pack, heated by the heating part, and then discharged, such that the media liquid may be efficiently heated in comparison with an apparatus in the related art in which a media liquid is heated while moving.

In addition, because the heating part for heating the media liquid is provided outside the refrigeration part, it is possible to reduce the amount of energy consumption in comparison with a case in which the heating part is provided in the refrigeration part.

In addition, according to the technical solution of the present application, the media liquid may be introduced into the pack by the media pump and the gas injection unit, such that the preset amount of media liquid may be supplied to the tube bag part.

### [Description of Drawings]

FIG. 1 is a schematic conceptual view illustrating a tube bag part, a pump part, a heating part, and a dispenser of a media liquid supply apparatus according to an embodiment of the present application.
FIG. 2 is a schematic perspective view illustrating a state in which some doors and some components of the media liquid supply apparatus according to the embodiment of the present application are excluded for ease of understanding.
FIGS. 3 to 8 are schematic operational views illustrating the tube bag part, the pump part, the heating part, and the dispenser of the media liquid supply apparatus according to the embodiment of the present application.
FIG. 9 is a schematic perspective view illustrating a state in which some doors of another example of the media liquid supply apparatus according to the embodiment of the present application are excluded.
FIG. 10 is a schematic front view illustrating a state in which some of the doors of the media liquid supply apparatus according to the embodiment of the present application equipped with two refrigeration parts are excluded.

### [Best Mode]

Hereinafter, embodiments of the present application will be described in detail with reference to the accompanying drawings so that those with ordinary skill in the art to which the present application pertains may easily carry out the embodiments. However, the present application may be implemented in various different ways and is not limited to the embodiments described herein. A part irrelevant to the description will be omitted in the drawings in order to clearly describe the present application, and similar constituent elements will be designated by similar reference numerals throughout the specification.

Throughout the specification of the present application, when one constituent element is referred to as being "connected to" another constituent element, one constituent element can be "directly connected to" the other constituent element, and one constituent element can also be "electrically connected to" the other element with other elements therebetween.

Throughout the specification of the present application, when one member is disposed "on", "at an upper side of", "at an upper end of", "below", "at a lower side of", or "at a lower end of" another member in the present specification of the present application, this includes not only a case where one member is brought into contact with another member, but also a case where still another member is present between the two members.

Throughout the specification of the present application, unless explicitly described to the contrary, the word "comprise" or "include" and variations, such as "comprises", "comprising", "includes" or "including", will be understood to imply the inclusion of stated constituent elements, not the exclusion of any other constituent elements.

The present application relates to a media liquid supply apparatus.

First, a media liquid supply apparatus (hereinafter, referred to as 'the present media liquid supply apparatus') according to an embodiment of the present application will be described.

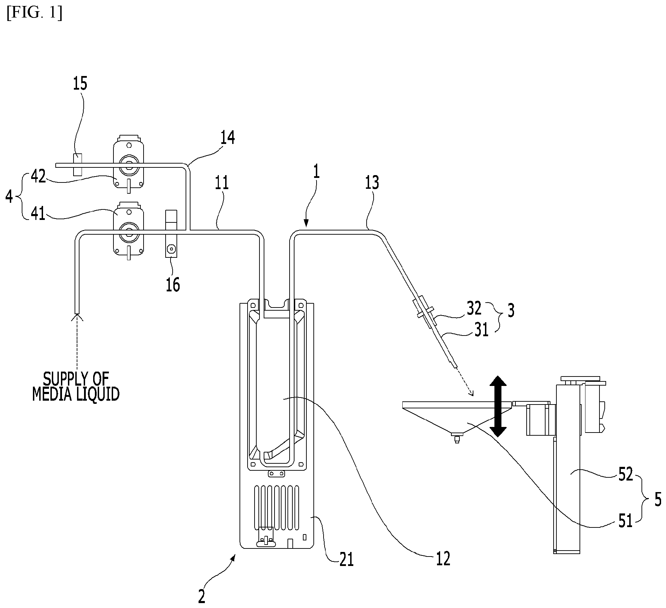

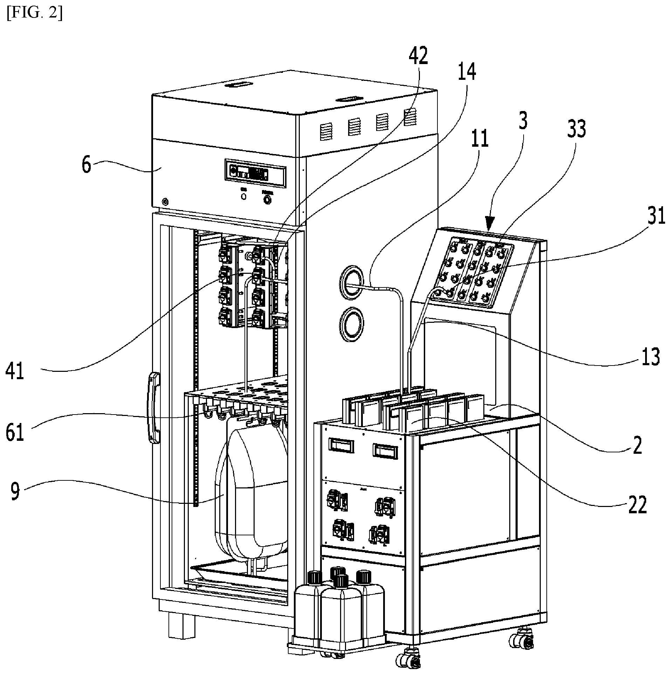

With reference to FIGS. 1 and 2, the present media liquid supply apparatus includes a tube bag part 1. The tube bag part 1 provides a passageway through which an introduced media liquid moves, an environment in which a temperature of the media liquid having passed through the passageway is raised, and a passageway through which the media liquid with the raised temperature is discharged. The tube bag part 1 includes an inflow tube part 11. The inflow tube part 11 defines an inflow passageway through which the media liquid is introduced, moved, and discharged.

In addition, with reference to FIG. 1, the tube bag part 1 includes a pack 12 configured to receive the media liquid discharged from the inflow tube part 11.

In addition, with reference to FIGS. 1 and 2, the tube bag part 1 includes a discharge tube part 13 configured to define a discharge passageway through which the media liquid discharged from the pack 12 is introduced, moved, and discharged.

In addition, with reference to FIGS. 1 and 2, the present media liquid supply apparatus includes a heating part 2 configured to heat the media liquid in the pack 12. The heating part 2 heats the media liquid passing through the tube bag part 1. The pack 12 may be mounted in the heating part 2, and the heating part 2 may heat the media liquid in the pack 12 by heating the pack. The heating part 2 will be described below in detail.

In addition, with reference to FIGS. 1 and 2, the present media liquid supply apparatus may include a dispenser 3. The dispenser 3 may be supplied with the media liquid from the tube bag part 1 and discharge the media liquid. The dispenser 3 may be supplied with the media liquid, which has been heated while passing through the tube bag part 1, and discharge the media liquid.

In addition, with reference to FIGS. 1 and 2, the present media liquid supply apparatus includes a pump part 4 configured to supply the media liquid to the pack 12 through the inflow tube part 11 and an auxiliary tube part 14. The pump part 4 may provide power for supplying the media liquid to the dispenser through the tube bag part 1 and moving the media liquid.

The pump part 4 may include a media pump 41 configured to move the media liquid through the inflow passageway. The media pump 41 may move a preset amount of media liquid so that the preset amount of media liquid is introduced into the pack. For example, the media pump 41 may introduce the preset amount of media liquid into the pack 12 through the inflow tube part 11. In this case, the preset amount may mean a capacity preset by a user and means the amount of media liquid that is discharged through the discharge tube part 13 after being heated in the pack 12.

In addition, the tube bag part 1 may include a gas pump 42 configured to provide an additional moving force to the media liquid that moves through the inflow passageway. The gas pump 42 may guide the discharge of media liquid to the pack by supplying gas to prevent the media liquid from stagnating in a section of the inflow tube part 11 between the media pump 41 and the pack 12. In addition, the gas pump 42 may supply the gas so that the media liquid in the pack 12 is discharged from the pack 12 through the discharge tube part 13.

Specifically, the gas pump 42 may supply the gas to the auxiliary tube part 14 that communicates with any one portion of the inflow tube part 11 between the media pump 41 and the pack 12. The gas pump 42 may be an air pump. In addition, a gas filter 15 may be provided in the auxiliary tube part 14. The gas filter 15 may be provided at a position at which the gas filter 15 may filter out foreign substances before the gas is introduced into the inflow tube part 11. The gas pump 42 may suck the gas and supply the gas to the auxiliary tube part 14, and the gas supplied to the auxiliary tube part 14 may be introduced into the inflow tube part 11. The gas filter serves as a filter configured to purify the gas sucked by the gas pump 42. In addition, for reference, the gas supplied by the gas pump 42 may be air.

In addition, the tube bag part 1 may include a sensor 16 configured to measure information on the media liquid passing through a section between a portion of the inflow tube part 11, where the media pump 41 is provided, and a portion of the inflow tube part 11 to which the auxiliary tube part 14 is connected. The information on the media liquid may include a flow rate, the amount, and a total amount of the media liquid that passes through a portion where the auxiliary tube part 14 is provided.

According to the above-mentioned description, the tube bag part 1 and the heating part 2 may operate as follows.

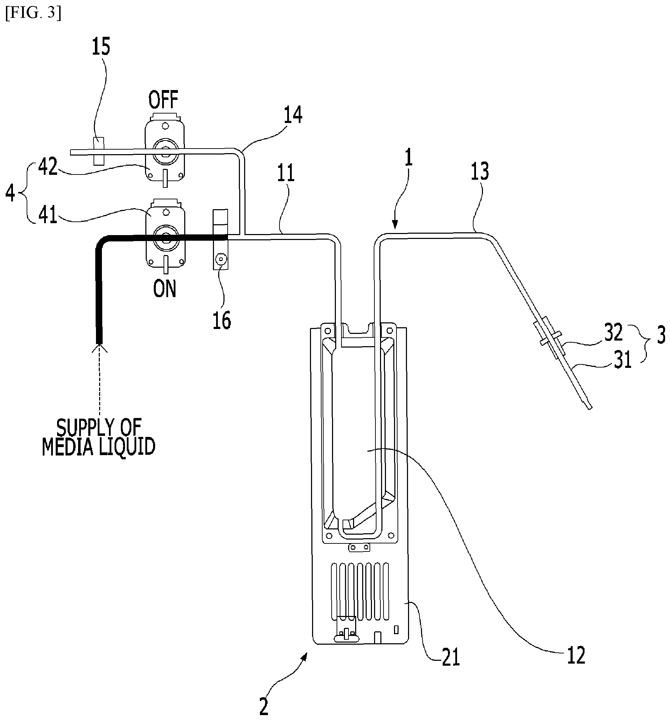

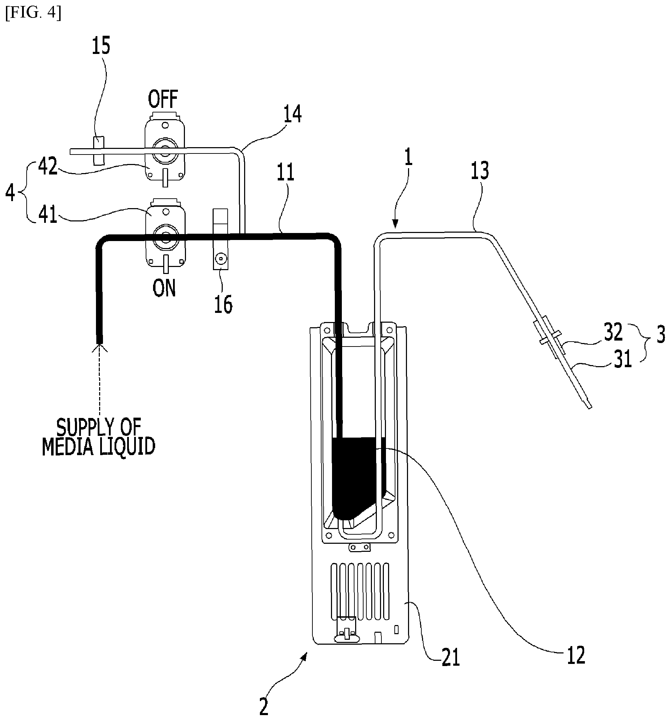

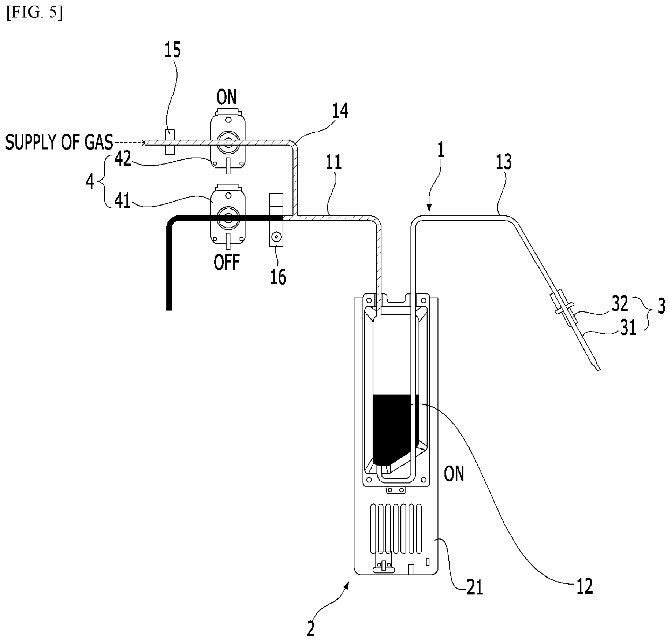

With reference to FIG. 3, the media pump 41 may operate to introduce (suck) the preset amount or more of media liquid into the inflow tube part 11. Therefore, with reference to FIG. 4, at least a part of the preset amount of media liquid may be introduced into the pack 12. In addition, with reference to FIG. 5, the sensor 16 may measure information (e.g., the amount of passage) on the media liquid that passes through the portion where the sensor 16 is provided. When the information measured by the sensor 16 indicates that the amount of passage of the media liquid passing through the portion where the sensor 16 is provided is a preset amount, the media pump 41 may stop operating, or the media pump 41 may operate to inject the gas into the pack 12 (the section between the pack 12 and the portion of the inflow tube part 11 where the sensor 16 is provided). Therefore, at least a part of the residual media liquid stagnating in the section between the pack 12 and the portion where the media pump 41 is provided (more specifically, the section between the pack 12 and the portion where the sensor 16 is provided) may be discharged to the pack 12. Therefore, the media liquid may be entirely injected in a preset amount into the pack 12.

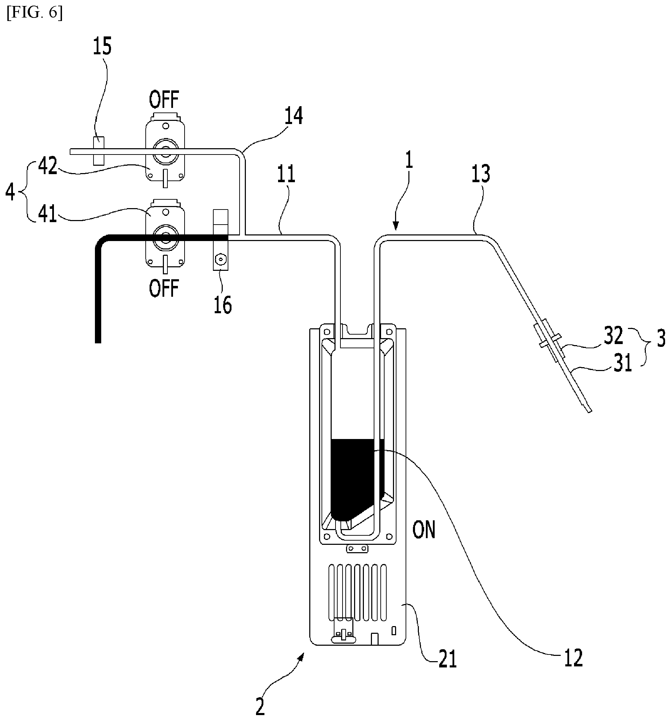

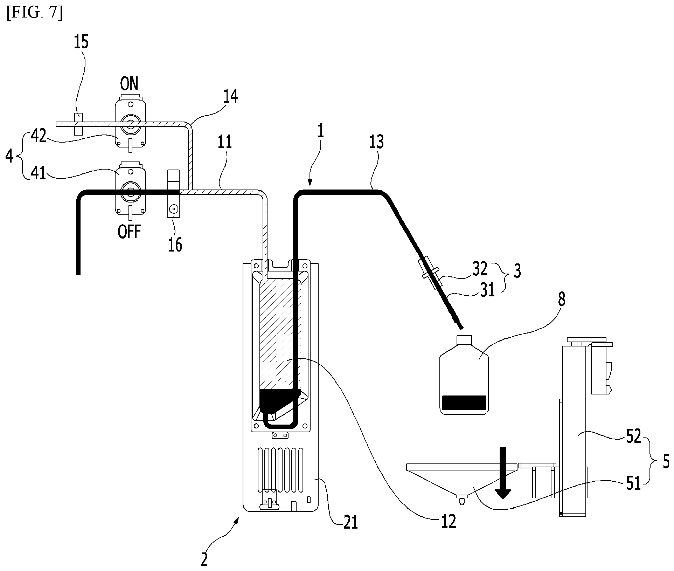

Thereafter, with reference to FIG. 6, the heating part 2 may heat the media liquid in a state in which the gas pump 42 and the media pump 41 have stopped operating. With reference to FIG. 7, when the heating process performed by the heating part 2 is ended, the gas pump 42 may supply the gas to the pack 12 to push the media liquid in the pack 12 toward the discharge passageway of the discharge tube part 13, and the media liquid introduced into the discharge tube part 13 may be discharged to the outside through a tube 31 of the dispenser 3 to be described below. In this case, the gas pump 42 may supply the gas so that the overall amount of media liquid in the pack 12 may be discharged to the outside through the tube 31. That is, the gas pump 42 may supply the gas so that the gas pushes the overall amount of media liquid in the pack 12, and the overall amount of media liquid is discharged to the outside through the tube 31. In this case, the discharged media liquid may be injected into a container 8 such as a flask or the like.

In addition, with reference to FIG. 1, the present media liquid supply apparatus may include a drain sink 5 configured to receive the media liquid from the tube 31 or the tube bag part 1. The drain sink 5 may include an accommodation part 51 configured to receive the media liquid, and a position adjustment rail structure 52 configured to adjust a position of the accommodation part 51 upward or downward. The accommodation part 51 may be provided below an end of the tube 31, at which the media liquid is discharged, so that the discharged media liquid may be dropped into the accommodation part 51. In addition, the accommodation part 51 may be disposed below the end of the tube 31 and moved upward or downward by the position adjustment rail structure 52.

In addition, when the media liquid is heated and then completely discharged from the pack 12 (the process related to FIGS. 3 to 7 may be completed when the media liquid in the pack 12 is completely discharged), the above-mentioned process (the process related to FIGS. 3 to 7) may be repeated again so that a subsequent preset amount of media liquid may be heated and discharged.

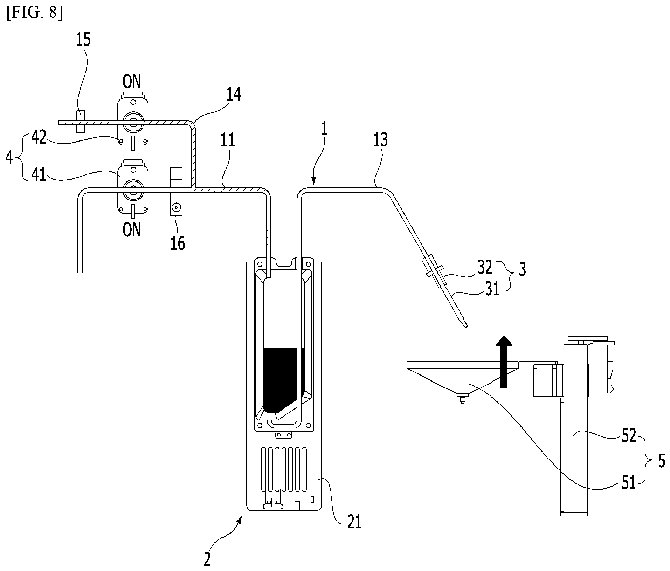

In addition, with reference to FIG. 8, the present media liquid supply apparatus may be reset. For example, the gas pump 42 may operate reversely, such that at least a part of the gas in the pack 12 or at least a part of the gas in the inflow tube part 11 may be discharged to the outside. In addition, the media pump 41 may operate reversely, such that the media liquid, which is positioned in the section between the portion of the inflow tube part 11 where the sensor 16 is provided and the portion of the inflow tube part 11 connected to the pack 12, or the media liquid in the pack 12 may be moved reversely (flow reversely) to the previous section of the portion where the sensor 16 is provided. Therefore, the state in FIG. 3 may be made. This resetting is performed when the media liquid is heated and then completely discharged from the pack 12 (the process related to FIGS. 3 to 7 may be completed when the media liquid in the pack 12 is completely discharged), such that the above-mentioned process may be performed again. Alternatively, the resetting may be performed in case that the process has been performed multiple times and need not be performed any further.

In addition, the drain sink 5 may accommodate at least a part of the discharged media liquid. For example, in case that the media liquid needs to be removed from the tube bag part 1 or the tube 31, the media liquid may be discharged from the tube 31, and the discharged media liquid may be dropped into the accommodation part 51 of the drain sink 5. This case may be applied when the media liquid is not accommodated in the separate container 8. In this case, the accommodation part 51 may be moved upward and disposed so that the media liquid discharged from the tube 31 is dropped into the accommodation part 51. In addition, usually, in particular, in case that the media liquid is discharged to the container 8 such as a flask, the container 8 may need to be disposed above the accommodation part 51 and communicate with the tube 31. Therefore, the accommodation part 51 may be in a state in which the accommodation part 51 is moved downward in order to prepare for a situation in which the media liquid is dropped (see FIG. 7). The drain sink 5 may prevent the media liquid from being discarded.

In addition, the present media liquid supply apparatus may include a refrigeration part 6. The refrigeration part 6 may be equipped with a media bag 9 having an internal space in which the media liquid is stored and accommodated. In addition, the refrigeration part 11 may maintain a preset temperature in the internal space.

The media liquid may be a solution required for cell culture. For example, the media liquid may be a cell culture medium. In addition, the media bag 9 may be provided as a plurality of media bags 9, and a plurality of solutions may be respectively stored in the plurality of media bags 9. For example, the plurality of solutions may include a cell culture medium, phosphate buffer saline (PBS), trypsin, Trypan Blue, and the like. In addition, the refrigeration part 11 may have both refrigerating and warming functions. A preset temperature of the refrigeration part 6 may be set by the user. For example, the preset temperature of the refrigeration part may be about 4 degrees Celsius when the media liquid is stored in refrigeration.

In addition, for example, the heating part 2 may increase the temperature of the media liquid by heating the media liquid so that the temperature of the media liquid becomes about 37 degrees Celsius similar to a body temperature of a human body. Alternatively, the media liquid may be heated to a cryogenic high temperature (e.g., 60 degrees Celsius), which may prevent proteins from spoiling. The temperature is not limited by the present application, and the media liquid may be heated to various temperatures.

In addition, with reference to FIG. 2, the media bag 9 may be disposed at a lower side of the internal space of the refrigeration part 6. In addition, at least a part of the inflow tube part 11 of the tube bag part 1, the media pump 41, and the sensor 16 may be disposed at an upper side of an internal space 11 of the refrigeration part 6. In addition, a mounting unit 61 may be provided at an upper side of a lower space of the refrigeration part 6, and the media bag 9 may be mounted on the mounting unit 61. In addition, the number of media bags 9 may be n, and the number of mounting units 61 may be n so that the n media bags 9 may be respectively mounted on the mounting units 61.

In addition, the number of tube bag parts 1 may be n, and the number of heating parts 2 may be n, such that the tube bag parts 1 and the heating parts 2 may correspond to the n media bags 9. For example, in case that eight media bags 9 are provided, eight tube bag parts 1 and eight heating parts 2 may be provided. In this case, eight inflow tube parts 11, eight pump parts 4, eight auxiliary tube parts 14, eight sensors 16, and the like may be provided at the upper side of the internal space 11 of the refrigeration part 6 while corresponding to the eight media bags 9.

In addition, the sensor 16 may include a capacitance sensor or the like. As described above, the sensor 16 may measure the amount of passage of the media liquid passing through the portion where the sensor 16 is provided. Therefore, as described above, when the amount of passage of the media liquid passing through the portion where the sensor 16 is provided is the preset amount, the media pump 41 may stop operating, the gas pump 42 may operate to inject the gas to the pack 12 (the section between the pack 12 and the portion of the inflow tube part 11 where the sensor 16 is provided), and the other operations may be performed.

In addition, when necessary, the sensor 16 may measure the amount of residual media liquid in the portion where the sensor 16 is provided. In this case, the sensor 16 may determine whether the movement of the media liquid through the inflow tube part 11 is ended on the basis of the amount of residual media liquid in the inflow tube part 11, and the media pump 41 and a gas injection unit 152 may operate on the basis of the amount of residual media liquid. For example, when the media liquid remains in the inflow tube part 11, the media pump 41 and the gas injection unit 34 may operate to move the media liquid. When the amount of residual media liquid is 0 within an error range, the media pump 41 and the gas injection unit 152 may stop operating. Therefore, the media liquid may be moved so that there is no residual media liquid in the inflow tube part 11 or the media bag 9.

In addition, a total amount of media liquid passing through the inflow tube part 11 may be calculated on the basis of the information measured by the sensor 16. Therefore, the amount of residual media liquid remaining in the media bag 9 may be calculated by comparing a total cumulative amount of passage of the media liquid measured by the sensor 16 with the amount of media liquid initially stored in the media bag 9, and the media pump 41 and the gas injection unit 152 may operate on the basis of the amount of residual media liquid. For example, when the residual media liquid is present, the media pump 41 and the gas injection unit 152 may operate to move the media liquid, as described above. When the amount of residual media liquid is 0 within an error range, the media pump 41 and the gas injection unit 152 may stop the movement of the media liquid. Therefore, the media liquid may be moved so that there is no residual media liquid in the media bag 9.

With reference to FIG. 1, the heating part 2 may include a heating block 21.

In addition, with reference to FIG. 2, the heating part 2 may include a housing 22 having an internal space opened upward, the heating block 21 may be disposed at a lower side of the internal space of the housing, and the pack 12 may be provided in the internal space and disposed above the heating block 21. In this case, the pack 12 may enter the internal space while being inserted downward into the internal space from above and thus be positioned on the heating block 21. The pack 12 may be detached upward and removed from the heating part 2.

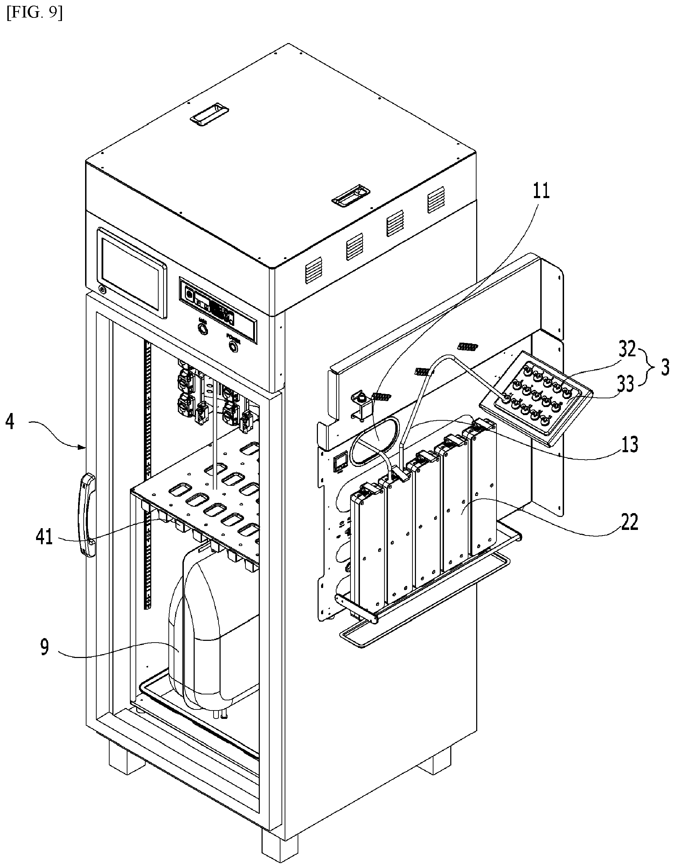

Alternatively, as another example, with reference to FIG. 9, the housing 22 may be provided in the form of a housing including an internal space and having a door capable of opening or closing the internal space. In this case, the door may be provided at a lateral side (see FIG. 4, a side directed in a 4 o'clock direction), and a lower end of the door is hingedly coupled to the housing, such that the door may be opened or closed in a lateral direction. In this case, when the door is opened, the pack 12 may be disposed in the internal space, and then the door may be closed.

In addition, with reference to FIG. 1, the pack 12 has a hollow portion formed therein. In addition, the inflow tube part 11 may communicate with an upper side of the hollow portion of the pack 12. In addition, a discharge tube 23 may be connected to a portion of the pack 12 positioned at a lowermost side and communicate with a lower side of the hollow portion. In addition, a hollow portion of a main tube part 21 may have a cross-section with a width that decreases downward. In addition, a lowermost end of the hollow portion of the main tube part 21 may be biased to one side based on a width direction (see FIG. 3, a 3 o'clock to 9 o'clock direction). The shape of the hollow portion may allow the pack 12 to heat the media liquid and discharge the entire media liquid through the discharge tube 31 so that the residual media liquid does not remain in the pack 12.

In addition, one or more heating parts 2 may be provided. For example, the number of heating parts 2 may be n, and the n packs 12 may be respectively mounted on the n heating part 2.

With reference to FIG. 2, the dispenser 3 may include the tubes 31. The tubes 31 may be provided as a plurality of tubes 31. In addition, the dispenser 3 may include a fixing plate part 33 configured to fix a position of the tube 31. In addition, the discharge tube part 13 may connect the pack 12 and the tube 31 and supply the media liquid, which is discharged from the pack 12, to the tube 31. For reference, the tube 31 and the discharge tube part 13 may be integrated. The tube 31 may be a part extending from the discharge tube part 13 and configured to define a portion through which the media liquid is discharged from the discharge tube part 13.

In addition, with reference to FIG. 1, the dispenser 3 may include a tube holder 32 that is a tube part that surrounds the tube 31. Because the tube holder 32 surrounds the tube 31, the tube 31 is supported by the tube holder 32, such that the media liquid may be easily discharged from the tube 31.

In addition, the number of tubes 31 may be m, and m may be a number equal to or larger than n. That is, the number of tubes 31 may be equal to or larger than the number (n) of media bags 9 or the number (n) of tube bag parts 2. In this case, among the m tubes 31, then tubes 31 may be respectively connected to the n tube bag parts 1, and the remaining tubes 31 may be connected to a separate room-temperature media liquid storage part.

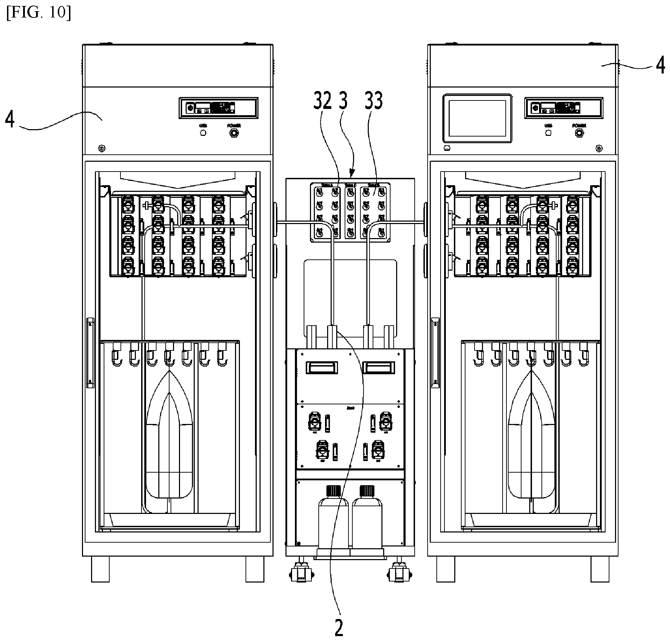

Although not illustrated in the drawings, the room-temperature media liquid storage part may be connected to the media bag 9 provided outside the refrigeration part 6 (at a room temperature) and supplied with the media liquid from the media bag 9. For example, with reference to FIG. 10, eight media bags 9 may be provided, eight dispensers 3 may be provided, eight tube bag parts 1 may be provided, and (eight +x) tubes 31 may be provided. In addition, for reference, in case that the number of refrigeration parts 6 is z, (z*n) tube bag parts 2 may be provided, and ((z*n) +x) tubes 31 may be provided. For example, in case that the number of refrigeration parts 6 is two, sixteen tube bag parts 1 may be provided, and twenty tubes 31 may be provided.

In addition, the heating part 2 and the dispenser 3 may be provided outside the refrigeration part 6. For example, with reference to FIGS. 1 and 2, the refrigeration parts 6 may be provided as a pair of refrigeration parts 6, and the heating part 2 may be positioned between the pair of refrigeration parts 6. Alternatively, with reference to FIG. 6, the single refrigeration part 6 may be provided, and the heating part 2 and dispenser 3 may be provided on an outer surface of the refrigeration part 6.

As described above, the present media liquid supply apparatus may store the media liquid in the refrigeration part 6 and automatically heat and discharge the media liquid stored in refrigeration. In addition, according to the present application, because the heating part 2 for heating the media liquid is provided outside the refrigeration part 6, it is possible to reduce the amount of energy consumption in comparison with a case in which the heating part 2 is provided in the refrigeration part 11.

In addition, the present application provides an automatic cell culture system including the present media liquid supply apparatus.

It will be appreciated that the embodiments of the present application have been described above for purposes of illustration, and those skilled in the art may understand that the present application may be easily modified in other specific forms without changing the technical spirit or the essential features of the present application. Therefore, it should be understood that the above-described embodiments are illustrative in all aspects and do not limit the present application. For example, each component described as a single type may be carried out in a distributed manner. Likewise, components described as a distributed type can be carried out in a combined type.

The scope of the present application is represented by the claims to be described below rather than the detailed description, and it should be interpreted that the meaning and scope of the claims and all the changes or modified forms derived from the equivalent concepts thereto fall within the scope of the present application.

## Claims

1. A media liquid supply apparatus comprising:
a tube bag part configured to provide a passageway through which an introduced media liquid moves, an environment in which a temperature of the media liquid having passed through the passageway is raised, and a passageway through which the media liquid with the raised temperature is discharged;
a heating part configured to heat the media liquid passing through the tube bag part;
a dispenser configured to be supplied with the heated media liquid and discharge the heated media liquid; and
a pump part configured to supply the media liquid to the dispenser through the tube bag part.

2. The media liquid supply apparatus of claim 1, wherein the tube bag part comprises:
an inflow tube part configured to define an inflow passageway through which the media liquid is introduced, moved, and discharged;
a pack configured to receive the media liquid discharged from the inflow tube part;
a discharge tube part configured to define a discharge passageway through which the media liquid discharged from the pack is introduced, moved, and discharged; and
an auxiliary tube part configured to supply air by allowing the inflow tube part to communicate with the outside.

3. The media liquid supply apparatus of claim 2, wherein the pump part comprises:
a media pump configured to move a preset amount of media liquid through the inflow tube part; and
a gas pump configured to guide a discharge of the media liquid to the pack by supplying a gas to prevent the media liquid from stagnating in a section between the pack and a portion of the inflow tube part where the media pump is provided.

4. The media liquid supply apparatus of claim 3, wherein the gas pump is configured to supply the gas so that the media liquid in the pack moves from the pack to the discharge tube part.

5. The media liquid supply apparatus of claim 4, wherein the tube bag part further comprises a sensor configured to measure information on the media liquid passing through the section between the portion of the inflow tube part where the media pump is provided and a portion of the inflow tube part to which the auxiliary tube part is connected.

6. The media liquid supply apparatus of claim 2, further comprising:
a refrigeration part configured to accommodate a media bag having an internal space in which the media liquid is accommodated,
wherein the inflow tube part receives the media liquid from the media bag.

7. The media liquid supply apparatus of claim 6, wherein the media bag is disposed at a lower side of the internal space of the refrigeration part, and the inflow tube part, the media pump, and the gas injection part are disposed at an upper side of the internal space of the refrigeration part.

8. The media liquid supply apparatus of claim 2, wherein the dispenser receives the media liquid from the discharge tube part and discharges the media liquid.

9. The media liquid supply apparatus of claim 2, wherein the heating part and the dispenser are provided outside the refrigeration part.

10. The media liquid supply apparatus of claim 8, wherein the dispenser comprises:
a tube; and
a fixing plate part configured to fix a position of the tube, and
wherein the discharge tube part connects the pack and the tube and supplies the media liquid, which is discharged from the pack, to the tube.
